Europäisches Patentamt
European Patent Office
Office européen des brevets

(11) Publication number: **0 367 189 A2**

# EUROPEAN PATENT APPLICATION

(21) Application number: 89120115.4

(22) Date of filing: 30.10.89

(51) Int. Cl.5 C07H 17/04 , A61K 31/70

(30) Priority: 31.10.88 US 264940

(43) Date of publication of application: 09.05.90 Bulletin 90/19

(84) Designated Contracting States: AT BE CH DE ES FR GB GR IT LI LU NL SE

(71) Applicant: BRISTOL-MYERS SQUIBB COMPANY
345 Park Avenue
New York, N.Y. 10154(US)

(72) Inventor: Ohnuma, Takeshi
1313, Zushi-machi
Machida-shi Tokyo(JP)
Inventor: Yamasaki, Tetsuro
5-1-401, Tsunashimadai
Kohoku-ku Yokohama(JP)
Inventor: Kamei, Hideo
3-30-6, Takaidohigashi
Suginami-ku Tokyo(JP)
Inventor: Naito, Takayuki
2657-45, Ohzenji
Asao-ku Kawasaki(JP)
Inventor: Saulnier, Mark
1225 Randolph Road
Middletown Connecticut 06457(US)

(74) Representative: Kinzebach, Werner, Dr. et al
Patentanwälte Reitstötter, Kinzebach und Partner Sternwartstrasse 4 Postfach 86 06 49
D-8000 München 86(DE)

## (54) Sugar phosphates and sulfonates of epipodophyllotoxin glucosides.

(57) The present invention relates to antitumor 4′-demethylepipodophyllotoxin glucosides having phosphate or sulfonate substituents on the sugar portion of the molecule.

EP 0 367 189 A2

# SUGAR PHOSPHATES AND SULFONATES OF EPIPODOPHYLLOTOXIN GLUCOSIDES

## BACKGROUND OF THE INVENTION

### 1. Field of the Invention

The present invention relates to 4′-demethylepipodophyllotoxin glucosides having various substituents on the sugar, to their use as antitumor agents and to pharmaceutical compositions containing them.

### 2. Background Art

4′-Demethylepipodophyllotoxin glucosides of formula (I) are antitumor agents derived from the naturally occurring lignan, podophyllotoxin (II). The method for their

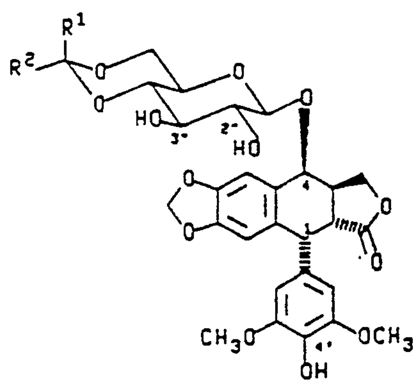

I

a: $R^1$=H; $R^2$=$CH_3$
b: $R^1$=H; $R^2$=2-thienyl

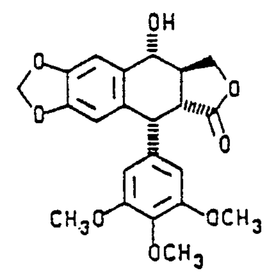

II

synthesis is described in US Patent 3,524,844 to Keller-Juslen et al. Among compounds of formula (I), etoposide (Ia) and teniposide (Ib) have been established as clinically useful against a variety of tumors including small cell lung, ovarian, testicular, breast, bladder, brain, non-lymphocytic leukemia, and Hodgkin's disease.

US Patents 4,547,567 and 4,716,221 disclose compounds of formula (III)

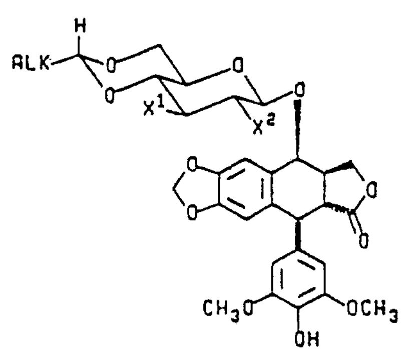

III

wherein one of $X^1$ and $X^2$ is OH, and the other is an amino, a monoalkylamino, or a dialkylamino group. These derivatives are said to exhibit high water solubility.

Seligman et al (Cancer Chemotherapy Reports Part I, 1975, 59:233-242) reported the preparation of podophyllotoxin phosphate disodium salt (IV). The phosphate, however, was not hydrolyzed by prostatic acid phosphatase and did not show reduced toxicity over the parent podophyllotoxin.

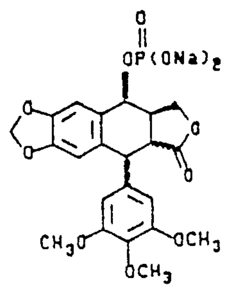

IV

## SUMMARY OF THE INVENTION

The present invention provides antitumor compounds of formula (V)

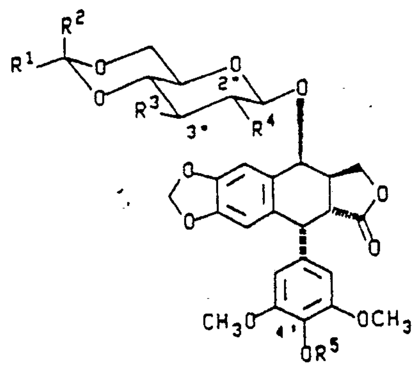

V

wherein $R^2$ is H and $R^1$ is selected from the group consisting of $(C_{1-10})$alkyl; $(C_{2-10})$alkenyl; $(C_{5-6})$cycloalkyl; 2-furyl; 2-thienyl; $(C_{6-10})$aryl; $(C_{7-14})$aralkyl; and $(C_{8-14})$aralkenyl wherein each of the aromatic rings may be unsubstituted or substituted with one or more groups selected from halo, $(C_{1-8})$alkyl, $(C_{1-8})$alkoxy, hydroxy, nitro, and amino; or $R^1$ and $R^2$ are each $(C_{1-8})$alkyl; or $R^1$ and $R^2$ and the carbon to which they are attached join to form a $(C_{5-6})$ cycloalkyl group; $R^3$ and $R^4$ are selected from the group consisting of OH, $-OSO_2R^6$, $-OSO_3H$ and pharmaceutically acceptable salts thereof, $-OP(X)(OH)_2$ and pharmaceutically acceptable salts thereof, and $-OP(X)(OR^7)_2$, wherein $R^6$ is hydrogen $(C_{1-5})$alkyl, phenyl, $(C_{1-5})$alkyl-substituted phenyl, or phenyl$(C_{1-5})$alkyl; $R^7$ is a phosphate protecting group; X is oxygen or sulfur with the proviso that $R^3$ and $R^4$ are not both OH; and $R^5$ is H or a phenol protecting group.

A preferred embodiment provides compounds of formula V wherein $R^4$ is OH and $R^3$ is as above-defined.

Another aspect of the invention provides compounds of formula (VI) and its pharmaceutically acceptable salts

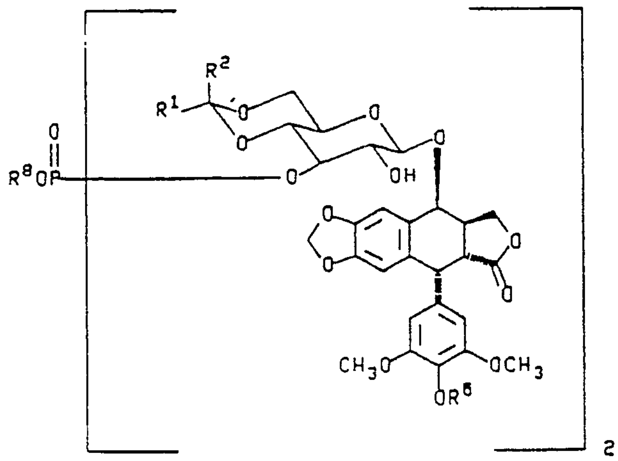

VI

wherein $R^1$, $R^2$, and $R^5$ are as defined above; and $R^8$ is H or a salt thereof, or $R^7$.

## DETAILED DESCRIPTION OF THE INVENTION

As used herein, unless otherwise specified, "phosphate", "phosphorylation" and related terms include "thiophosphate", "thiophosphorylation" etc; "hal", "halide", and halogen refer to chlorine, bromine, and iodine; "alkyl" refers to linear and branched carbon chains; and the notation "-L" signifies a leaving group, such as chloride, that is commonly encountered in esterification reactions.

The starting materials, 4′-demethylepipodophyllotoxin glucosides (I), may be prepared by the procedures described in US Patent 3,524,844 which is hereby incorporated by reference. In order to effectively derivatize the hydroxyl groups on the sugar portion, it is necessary to first block the reactive phenol group. Protection of the 4′-phenol group of (I) may be achieved by using known procedures which include, for example the formation of ethers, acetals or esters. The selection of the phenol protecting group is not specifically restricted and may be any of the conventional protecting groups known in the art. The choice of protecting group, and methods for blocking and deblocking the phenol group are documented in treatises such as "Protective Groups in Organic Chemistry", J.F.W. McOmie, Ed., Plenum Press, 1973. Examples of suitable phenol protecting group include, but are not limited to, t-butyl, benzyl, 2-tetrahydropyranyl, 2,2,2-trichloroethoxycarbonyl, and benzyloxycarbonyl, the last being the preferred.

The sugar hydroxyl groups of a thus protected 4′-demethylepipodophyllotoxin glucoside (I) may then be converted into phosphates, sulfates or sulfonates of the present invention by conventional methods known in the literature. The reactions are usually non-regiospecific and result in a mixture of 2″-, 3″-, and 2″,3″-bis derivatized products and in the case of phosphorylation the phosphate dimer of formula (VI) as well. The product mixture may be separated into the individual components by using art-recognized techniques such as column chromatography. It is not critical at what point in the reaction sequence the mixture of reaction products is separated.

Phosphorylation may be achieved by reacting a 4′-phenol protected (I) with phosphoryl halide, P(X)-$(hal)_3$, in an inert organic solvent, e.g. methylene chloride or acetonitrile, and in the presence of an acid acceptor, preferably a tertiary amine base such as pyridine or diisopropylethylamine. The resulting mixture of intermediate dichlorophosphates may be hydrolyzed in situ to provide phosphate monoesters; and if the hydrolysis is carried out in the presence of a base such as sodium bicarbonate and the like, the corresponding salt of the phosphate monoester is obtained. The 4′-phenol protecting group is subsequently removed to yield phosphates of the present invention.

The intermediate dichlorophosphates may also be converted into phosphate triesters upon treatment with an alcohol of the formula $R^7OH$, wherein $R^7$ is a phosphate protecting group. Phosphate protecting groups include, but are not limited to, $(C_{1-5})$ alkyl optionally substituted with one or more halogen atoms; phenyl optionally substituted with one or more groups selected from $(C_{1-5})$ alkyl, halogen, and nitro; and phenyl$(C_{1-5})$alkyl wherein the phenyl ring is optionally substituted as above. The phosphate triester may also

be obtained by treating 4'-phenol protected (I) with a a compound of formula $(R'O)_2P(X)$-L in an inert organic solvent in the presence of an acid acceptor. The R' group on the phosphate triester may be optionally removed to provide the phosphate monoester which may be readily converted into a pharmaceutically acceptable salt by treating it with a base such as sodium bicarbonate. Thus, the R' group on the phosphate triester may be chosen such that it may be removed simultaneously with the phenol protecting group or one may be selectively removed without affecting the other.

Sulfates may be prepared by treating 4'-phenol protected (I) with sulfur trioxide or a complex thereof such as sulfur trioxide-pyridine complex in an inert organic solvent at ambient temperature. The phenol group is removed using conventional methods well known in the art to provide the product. A sulfate derivative may be readily converted to its pharmaceutically acceptable salts by contacting it with a source of the desired cation.

Sulfonates of the present invention may be similarly obtained by reacting a 4'-phenol protected (I) with a compound of the formula $R^5SO_2$-L. The reaction is performed in an inert organic solvent and in the presence of an acid acceptor at a temperature from room temperature to about -20 °C. Removal of the phenol protecting group provides the final product.

It is to be understood that synthesis of compounds of the present invention is not limited to the procedures and reagents outlined above, but may include other methods capable of introducing phosphate or sulfonate group on to the sugar portion of the 4'-demethylepipodophyllotoxin glucosides. The reaction conditions will of course vary with the choice of starting materials but may be ascertained by a skilled artisan without undue experimentation.

Compounds of the present invention may be used to inhibit growth of malignant tumors in mammalian hosts including human. Some of the compounds, particularly the phosphates and sulfates, may be considered as prodrugs of 4'-demethylepipodophyllotoxin glucosides and as such are converted to the active parent drug in vivo by factors either endogenous in the host or exogenously supplied. These prodrugs may be administered alone or in combination with an agent capable of releasing the active form in vivo, for example enzymes capable of cleaving the phosphate or sulfate group. Examples of suitable enzymes include phosphatases such as alkaline phosphatase and sulfatases such as aryl sulfatase. The enzyme may be linked to a tumor specific antibody to form an enzyme-antibody conjugate which may then be administered to a tumor bearing host prior to, concurrent with, or after the administration of the prodrug. The prodrug plus antibody-enzyme conjugate is the preferred approach in administering the sulfate prodrugs of the present invention. Antibody-enzyme conjugates, methods for their preparation and methods for their use are disclosed in detail in our co-pending patent application serial number 211,301 filed June 29, 1988 and its parent and grandparent applications 161,068 filed February 26, 1988 and 081,382, filed August 4, 1987, respectively. rnese applications are hereby incorporated by reference.

## BIOLOGICAL ACTIVITY

Representative compounds of the present invention were evalnated for anti tumor activity against murine transplantable P388 leukemia. Five-week old female $CDF_1$ mice were inoculated intraperitoneally with 0.4 ml of diluted ascitic fluid containing $10^6$ lymphocytic leukemia P388 cells. Test compounds were administered intraperitoneally as a single dose on day 1 and animals were observed for 45 days. The percent increase of median survival time (MST) of treated animals over that of untreated control animals was determined and reported as % T/C. Compounds showing % T/C values of 125 or greater are considered to have significant antitumor activity. Table I presents the results of the in vivo evaluation; only the maximum % T/C and the dose giving the maximum effects are reported.

Table I.

| Antitumor activity against P388 leukemia | | |
|---|---|---|
| Compound | Dose(mg kg day) | %T C of MST |
| Etoposide | 120 | 250 |
| XI | 120 | 185 |
| XII | 30 | 120 |
| XIII | 30 | 125 |
| XVII | 60 | 195 |
| XVIII | 30 | 115 |
| XIX | 30 | 100 |
| XXIII | 120 | 175 |
| XXIV | 60 | 130 |
| XXVI | 30 | 130 |
| XXVII | 120 | 105 |
| XXVIII | 10 | 105 |

Cytotoxicity of compounds of the present invention was studied using B16 melanoma cells. B16 melanoma cells were grown to the logarithmic phase in the enriched Eagle minimum essential medium supplemented with fetal calf serum (10%) and kanamycin (60 mcg/ml). The tumor cells were harvested and implanted into wells of the 96-well microtiter plate with test materials at the inoculum size of 3.0 x $10^4$ cells/ml. They were incubated at 37°C in humidified atmosphere of 5% $CO_2$ and 95% air for 72 hours. The cytotoxic activity against the above cell line was determined colorimetrically at 540 nm after staining viable cells with neutral red and is reported in terms of the concentration that inhibits 50% of cell growth ($IC_{50}$). $IC_{50}$ values of higher than 100 μg/ml are generally considered as indicating lack of cytotoxic activity.

Table II.

| In vitro cytotoxicity against B16 melanoma. | |
|---|---|
| Compound | $IC_{50}$ (μg/ml) |
| etoposide | 0.21 |
| XI | 2.2 |
| XII | 20 |
| XIII | 37 |
| XVII | 0.07 |
| XVIII | 5.4 |
| XIX | 30 |
| XXIII | 0.32 |
| XXIV | 29 |
| XXVI | 8.0 |

The present invention provides a method for inhibiting mammalian tumors which comprises administering an effective tumor-inhibiting dose of an antitumor compound of formula V or VI to a tumor bearing host. For this purpose, the drug may be administered by conventional routes including, but not limited to, intravenous, intramuscular, intratumoral, intraarterial, intralymphatic, and oral. In certain cases, the drug may be administered in combination with an antibody-enzyme conjugate which can activate the drug in vivo.

A further aspect of the present invention provides a pharmaceutical composition which comprises a compound of formula V or VI and a pharmaceutically acceptable carrier. The antitumor composition may be

made up of any pharmaceutical form appropriate for the desired route of administration. Examples of such compositions include solid compositions for oral administration such as tablets, capsules, pills, powders granules, liquid compositions for oral administration such as solutions, suspensions, syrups or elixirs and preparations for parenteral administration such as sterile solutions, suspensions or emulsions. They may also be manufactured in the form of sterile solid compositions which can be dissolved in sterile water, physiological saline or some other sterile injectable medium immediately before use.

Optimal dosages and regimens for a given mammalian host can be readily ascertained by those skilled in the art. It will, of course, be appreciated that the actual dose used will vary according to the particular composition formulated, the particular compound used, the mode of application and the particular site, host and disease being treated. Many factors that modify the action of the drug will be taken into account including age, weight, sex, diet, time of administration, route of administration, rate of excretion, condition of the patient, drug combinations, reaction sensitivities and severity of the disease.

The following examples are for illustrative purposes only and should not be construed as limiting the scope of the invention which is defined solely by the Claims appended to this application.

Example 1

a. Preparation of 4′-O-benzyloxycarbonyl etoposide (VII)

Benzylchloroformate (1.98 ml, 15 mmol) was added over a period of 30 minutes to a mixture of etoposide (5.88 g, 10 mmol) and pyridine (10 Ml) in methylene chloride (100 ml) kept at -15°C. The mixture was stirred at -15°C for an additional hour, washed successively with 5% HCl, aqueous sodium bicarbonate, and water, and then dried over anhydrous sodium sulfate. The solvent was evaporated to give 8.05 g of the crude product which was purified on a silica gel column (5% methanol-methylene chloride) to give 6.93 g (96%) of 4′-O-benzyloxycarbonyl etoposide as a colorless semi-solid. M.P. 152-155°C
IR$\nu_{max}$ (Nujol) cm$^{-1}$: 3200-3600 (OH), 1760 (lactone & 4′-O-benzyloxy carbonyl), 1600 (aromatic).
$^1$H NMR (60 MHz, $CDCl_3$) δ 7.36 (5H, s, $OCO_2CH_2Ph$), 6.81 (1H, s, 5-H), 6.50 (1H, s, 8-H), 6.25 (2H, s, 2′- and 6′-H), 5.94 (2H, br.s, O-$CH_2$-O), 5.23 (2H, s, -$OCO_2CH_2Ph$), 4.89 (1H, d, J=4 Hz, 4-H), 3.66 (6H, s, 3′,5′-$OCH_3$), 2.8-3.0 (2H, m, 2″,3″-OH, $D_2O$ exchanged), 1.38 (3H, d, J=5 Hz, 7″-$CH_3$).

b. Phosphorylation of 4′-O-benzyloxycarbonyl etoposide.

Phosphorous oxychloride (280 µl, 3 mmol) was slowly added to a cold solution (0°C) of 4′-O-benzyloxycarbonyl etoposide (1.511 g, 2.1 mmol) in dry methylene chloride (10 ml) and pyridine (10 ml) and the mixture was stirred at 0°C for 45 minutes. Benzyl alcohol (620 µl, 6 mmol) was then added and the reaction mixture was allowed to warm to room temperature and after being stirred for two hours, it was diluted with methylene chloride (50 ml), washed with water, and dried over sodium sulfate. The solvent was evaporated in vacuo to give 2.40 g of crude semi-solid containing three components (silica gel TLC: $R_f$ 0.66, 0.54, and 0.46) which were separated by silica gel chromatography (1% methanol-methylene chloride). Fractions showing a spot of Rf 0.66 were combined and evaporated in vacuo to give 435 mg (21%) of 4′-benzyloxycarbonyl etoposide 3″-phosphate dibenzyl ester (VIII). Similarly, fractions of $R_f$ 0.54 and 0.46 afforded 352 mg (21%) of bis(4′-benzyloxycarbonyletoposide 3″-O-)phosphate benzyl ester (IX), and 262 mg (13%) of 4′-benzyloxycarbonyletoposide 2″-phosphate dibenzyl ester (X), respectively.
IR $\nu_{max}$ (Nujol) cm$^{-1}$:
VIII: 3300 (br), 1760, 1600.
IX: 3300 (br), 1760, 1600.
X: 3350, 1760, 1600.

c. Preparation of disodium 3″- and 2″-etoposide phosphates and sodium bis(etoposidyl-3″-)phosphate.

A solution of 4′-benzyloxycarbonyl etoposide 3″-phosphate dibenzyl ester (270 mg) in methanol (20 ml) and ethyl acetate (20 ml) containing 10% palladium on carbon (50 mg) was hydrogenated at 1 atm for 1 hour. The catalyst was removed by filtration and the filtrate was concentrated in vacuo to give 188 mg of a semi-solid, which was triturated with ethyl acetate to give 145 mg (79%) of etoposide 3″-dihydrogen

phosphate as a light brown powder. A 135 mg sample of the above product was dissolved in aqueous sodium bicarbonate (33.6 mg in 2 ml water) and lyophilized to give 140 mg of disodium etoposide 3″-phosphate (Compound XI) as a gray powder: estimated purity:95% by HPLC (LiChrosorb RP-18, 70% MeOH-$H_2O$). M.P. 262-263 °C (dec.)
IR $\nu_{max}$ (Nujol) $cm^{-1}$: 3377 (br), 1762, 1615.
UV $\lambda_{max}$ (MeOH) nm ($\epsilon$): 238 (sh, 13,000), 285 (4,010).
SIMS m/z: 713 $(M+H)^+$.

According to the above procedure, 97 mg of 4′-benzyloxycarbonyl etoposide 2″-phosphate dibenzyl ester was hydrogenated to give 70 mg (ca. 100%) of disodium etoposide 2″ -phosphate (Compound XII) as a gray powder. Estimated purity: 95% by HPLC.
M.P. 255-257 °C (dec.).
IR $\nu_{max}$ (KBr) $cm^{-1}$: 3460, 1772, 1615.
UV $\lambda_{max}$ nm ($\epsilon$): 240 (sh, 12,600), 284 (3,740).
SIMS m/z: 713 $(M+H)^+$, 735 $(M+Na)^+$

Similarly, 135 mg of bis (4′-benzyloxycarbonyl etoposide-3″- )phosphate benzyl ester was hydrogenated to give 60 mg (58%) of sodium bis(etoposide-3″-)phosphate (Compound XIII) as a light gray powder. Estimated purity: 95% by HPLC.
M.P. 265-266 °C (dec.)
IR $\nu_{max}$ (KBr) $cm^{-1}$: 3460, 1772, 1620.
UV $\lambda_{max}$ (MeOH) nm ($\epsilon$): 240 (sh, 24,600), 285 (7,730).
SIMS m/z: 1262 $(M+H)^+$, 1285 $(M+Na+1)^+$.

## Example 2

a. Tosylation of 4′-benzyloxycarbonyl etoposide.

4-Dimethylamino pyridine (200 mg) and tosyl chloride (870 mg, 4.6 mmol) were added to a solution of 4′-benzyloxycarbonyl etoposide (product of Example 1, Step a, 2.2 g, 3 mmol) in dry pyridine (50 ml) at 0 °C, and the mixture was stirred for 3 days at room temperature. Molecular sieve (4Å) and additional tosyl chloride (870 mg, 4.6 mmol) were added to the mixture and stirring continued for one week at room temperature. At the end of the period the precipitate was filtered off; the filtrate was diluted with $CH_2Cl_2$, washed with water, 5% HCl, aq. $NaHCO_3$, and water, dried over anhyd. sodium sulfate and then concentrated *in vacuo* to give the crude solid (2.35 g) containing three components (silica gel TLC: Rf 0.8, 0.6 and 0.4), which was separated by silica gel column chromatography (2% MeOH-$CH_2Cl_2$). Fractions showing a spot of Rf 0.8 were combined and evaporated *in vacuo* to give 602 mg (19%) of 4′-benzyloxycarbonyl etoposide 2″,3″-ditosylate (XIV) as a colorless powder. Similarly fractions of Rf 0.6 and Rf 0.4 afforded 690 mg (26%) of 4'benzyloxycarbonyl etoposide 3″-tosylate (XV) and 156 mg (6%) of 4′-benzyloxycarbonyl etoposide 2″-tosylate (XVI) as hygroscopic colorless powder, respectively.

### 4′-benzyloxycarbonyl etoposide 3″-tosylate (XV)

M.P. 145-148 °C.

| | | | |
|---|---|---|---|
| Anal Calcd for $C_{44}H_{44}O_{17}S \cdot 3H_2O$: | C 56.77, | H 5.41, | S 3.44. |
| Found: | C 56.84, | H 4.91, | S 3.90. |

### 4′-benzyloxycarbonyl etoposide 2″-tosylate (XVI)

M.P. 135-139 °C.

| | | | |
|---|---|---|---|
| Anal Calcd for $C_{44}H_{44}O_{17}S \cdot 3H_2O$: | C 56.77, | H 5.41, | S 3.44. |
| Found: | C 56.61, | H 4.89. | S 3.70. |

4′-benzyloxycarbonyl etoposide 2″,3″-ditosylate (XIV)

M.P. 149-152° C.

b. Preparation of etoposide 3″- and 2″-tosylates and 2″,3″-ditosylate.

A stirred solution of 4′-benzyloxycarbonyl etoposide 3″-tosylate (47 mg, 0.05 mmol) in ethanol-acetone (4:1, 2.5 ml) was hydrogenated at 1 atm for 1 hour in the presence of 10% palladium on carbon (40 mg). The catalyst was filtered off and the filtrate was concentrated in vacuo to give etoposide 3″-tosylate (Compound XVII) (42 mg, ca 100%) as a colorless powder which was then recrystallized from methanol to give the product as colorless crystals. Estimated purity: 90% by HPLC.
M.P. 175-178° C
IR $\nu_{max}$ (KBr) $cm^{-1}$ 3500 (br), 1780, 1600.
UV $\lambda_{max}$ (MeOH) nm ($\epsilon$) 245 (sh, 10,260), 284 (3,900).

| | | | |
|---|---|---|---|
| Anal Calcd for $C_{36}H_{38}O_{15}S$: | C 58.21, | H 5.16, | S 4.32. |
| Found: | C 57.80, | H 5.14, | S 4.43. |

According to the above procedure, 100 mg (0.39 mmol) of 4′-benzyloxycarbonyl etoposide 2″-tosylate was hydrogenated to give 114 mg (ca 100%) of etoposide 2″-tosylate (Compound XVIII) colorless powder which was recrystallized from methanol to provide the product as hygroscopic crystals. Estimated purity 90% by HPLC.
M.P. 210-212° C.
IR $\nu_{max}$ (KBr) $cm^{-1}$ 3500 (br), 1780, 1600.
UV $\lambda_{max}$ (MeOH) nm ($\epsilon$) 243 (sh, 4,560), 282 (1,450).

| | | | |
|---|---|---|---|
| Anal Calcd for $C_{36}H_{38}O_{15}S \cdot H_2O$: | C 56.83, | H 5.30, | S 4.21. |
| Found: | C 56.87, | H 5.37, | S 4.19. |

Similarly, 80 mg (0.08 mmol) of 4′-benzyloxycarbonyl etoposide 2″,3″-ditosylate was hydrogenated to give 72 mg (ca 100%) of etoposide 2″,3″-ditosylate (Compound XIX) as a colorless powder which was recrystallized from methanol to provide the product as colorless crystals. Estimated purity 90% by HPLC.
M.P. 169-171° C.
IR $\nu_{max}$ (KBr) $cm^{-1}$ 3400 (br), 1770, 1600.
UV $\lambda_{max}$ (MeOH) nm ($\epsilon$) 245 (sh, 11,750), 284 (3,840).

Example 3

a. Mesylation of 4′-benzyloxycarbonyl etoposide.

Mesyl chloride (270 µl, 2.8 mmol) was added to a solution of 4′-benzyloxycarbonyl etoposide (1.00 g, 1.4 mmol) in dry pyridine (50 ml) and the mixture was stirred overnight at room temperature. The reaction mixture was diluted with ethyl acetate; washed with water, 5% HCl, aqueous sodium bicarbonate, and water; dried over sodium sulfate, and then filtered. The filtrate was concentrated in vacuo to give a crude solid (1.4 g) containing three components (silica gel TLC: Rf 0.51, 0.44 and 0.40) which were separated by silica gel column chromatography (1% methanol-methylene chloride). Fractions showing a spot of Rf 0.51 were

combined and evaporated in vacuo to give 440 mg (36%) of 4′-benzyloxycarbonyl etoposide 2″,3″-dimesylate (XX). Similarly, fractions of Rf 0.44 and 0.40 afforded 314 mg (32%) of 4′-benzyloxycarbonyl etoposide 3″-mesylate (XXI) and 248 mg (26%) of 4′-benzyloxycarbonyl etoposide 2-mesylate (XXII), respectively as colorless powders.

4′-benzyloxycarbonyl etoposide 3″-mesylate (XXI)

M.P. 167-170 °C.

| | | | |
|---|---|---|---|
| Anal Calcd for $C_{33}H_{40}O_{17}S \cdot 1\ 2H_2O$: | C 56.36, | H 4.98, | S 3.96. |
| Found: | C 56.23, | H 5.06, | S 4.14. |

4′-benzyloxycarbonyl etoposide 2″-mesylate (XXII)

M.P. 159-161 °C. Hygroscopic.

| | | | |
|---|---|---|---|
| Anal Calcd for $C_{38}H_{40}O_{17}S \cdot 3H_2O$: | C 55.13, | H 5.11, | S 3.87. |
| Found: | C 55.29, | H 5.00, | S 4.00. |

4′-benzyloxycarbonyl etoposide 2″,3″-dimesylate

M.P. 226-228 °C.

| | | | |
|---|---|---|---|
| Anal Calcd for $C_{39}H_{42}O_{19}S_2 \cdot 1\ 2H_2O$: | C 52.76, | H 4.77, | S 7.22. |
| Found: | C 52.78, | H 4.77, | S 7.31. |

b. Preparation of etoposide 3″- and 2″-mesylates

A stirred solution of 4′-benzyloxycarbonyl etoposide 3″-mesylate (60 mg, 0.07 mmol) in ethanol-acetone (4:1, 2.5 ml) was hydrogenated at 1 atm for 1 hour in the presence of 10% palladium on carbon (30 mg). After the catalyst was filtered off, the filtrate was concentrated in vacuo to provide etoposide 3″-mesylate (Compound XXIII) as a colorless powder. Estimated purity: 90% by HPLC.
M.P. 226-229 °C.
IR $\nu_{max}$ (KBr) $cm^{-1}$ 3500 (br), 1770, 1610.
UV $\lambda_{max}$ (MeOH) nm ($\epsilon$) 740 (sh, 12,900), 286 (3,950).

Similarly, 30 mg (0.04mmol) of 4′-benzyloxycarbonyl etoposide 2″-mesylate was hydrogenated to give 23 mg (94%) of etoposide 2″-mesylate (Compound XXIV) as a colorless powder. Estimated purity 95% by HPLC.
M.P. 379-181 °C.
IR $\nu_{max}$ (KBr) $cm^{-1}$ 3500 (br), 1770, 1620.
UV $\lambda_{max}$ (MeOH) nm ($\epsilon$) 240 (sh, 12,200), 285 (3,950).

Example 4

A mixture of 4′-benzyloxycarbonyl etoposide (350 mg, 0.5 mmol) and diphenylphosphonyl chloride (322 mg, 1.2 mmol) was stirred at room temperature for two days; diluted with methylene chloride (50 ml); washed with water, 10% HCl and aqueous sodium bicarbonate; and then dried over anhydrous sodium

sulfate. The solvent was evaporated in vacuo to give 4′-benzyloxycarbonyl etoposide 3″-diphenyl phosphate (XXV) as a crude semi-solid (290 mg). A stirred solution of this semi-solid (190 mg) was hydrogenated at 1 atm with 10% palladium on carbon as the catalyst. After the catalyst was filtered off, the filtrate was concentrated in vacuo to give 131 mg of a crude semi-solid which was then chromatographed on a silica gel column (1 methanol-methylene chloride) to afford 59 mg (20%) of etoposide 3″-diphenyl phosphate (compound XXVI). Estimated purity: 80% by HPLC.

M.P. 126-130° C.

IR $\nu_{max}$ (Nujol) $cm^{-1}$ 3350, 1770, 1600.

FAB MS m/z 820 ($M^+$).

Example 5

Preparation of sodium 3″- and 2″-etoposide sulfates

To a solution of 4′-benzyloxycarbonyletoposide (500 mg, 0.69 mmol) in dry pyridine (20 ml) under argon was added sulfur trioxide-pyridine complex (330 mg, 2.07 mmol). After stirring at room temperature for three hours, an additional sulfur trioxide-pyridine complex (250 mg, 1.59 mmol) was added and stirred at room temperature for 15 hours. The mixture was concentrated in vacuo below 40° C to give a crude oil (1.2 g), which was chromatographed on a silica gel column (2% $MeOH-CH_2Cl_2$) to afford a mixture of the 2″- and 3″-sulfates (411 mg). A stirred solutin of this mixture (400 mg, 0.49 mmol) in EtOH-acetone (4:1, 15 ml) was hydrogenated with 10% palladium on carbon (400 mg) at 1 atm. The catalyst was removed by filtration and the filtrate was concentrated in vacuo to give 370 mg of a mixture of the regioisomers, which was separated by $C_{18}$-reverse phase column chromatography to afford 52 mg (16%) of the 3″-sulfate (XXVII), 107 mg (32%) of the 2″-sulfate (XXVIII) and 138 mg of these mixtures. The 3″-sulfate was passed through a column of Dowex 50W ($Na^+$) resin with water as eluant and the water-soluble fractions were lyophilized to give the corresponding sodium salt of XXVII as colorless powder. Similarly, the sodium salt of XXVIII was obtained as colorless powder.

XXVII: MP 223-225° C. Estimated purity: 90% by HPLC.

IR $\nu_{max}$ (KBr) $cm^{-1}$ 3440, 1770, 1620. UV $\lambda_{max}$ (MeOH) nm ($\epsilon$) 240 (sh, 11,600), 284 (3,670). SIMS m/z 691 $(M+H)^+$, 713 $(M+Na)^+$.

XXVIII: MP 246-247° C. Estimated purity: 95% by HPLC. IR $\nu_{max}$ (KBr) $cm^{-1}$ 3450, 1770, 1620. UV $\lambda_{max}$ (MeOH) nm ($\epsilon$) 240 (sh, 11,200), 285 (3,650). SIMS m/z 691 $(M+H)^+$, 713 $(M+Na)^+$.

The structures of compounds prepared in Examples 1-5 are given below:

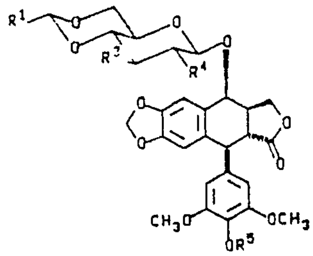

V

| Ex. | Compound | $R^1$ | $R^3$ | $R^4$ | $R^5$ |
|---|---|---|---|---|---|
| 1a | VII | $CH_3$ | -OH | -OH | CBZ* |
| 1b | VIII | $CH_3$ | $(BzO)_2PO_2$- | -OH | CBZ |
| 1b | X | $CH_3$ | OH | $(BzO)_2PO_2$- | CBZ |
| 1c | XI | $CH_3$ | $(NaO)_2PO_2$- | -OH | H |
| 1c | XII | $CH_3$ | - OH | $(NaO)_2PO_2$- | H |
| 2a | XIV | $CH_3$ | $pCH_3$-$C_6H_4$-$SO_3$- | $pCH_3$-$C_6H_4$-$SO_3$- | CBZ |
| 2a | XV | $CH_3$ | $pCH_3$-$C_6H_4$-$SO_3$- | OH | CBZ |
| 2a | XVI | $CH_3$ | -OH | $pCH_3$-$C_6H_4$-$SO_3$- | CBZ |
| 2b | XVII | $CH_3$ | $pCH_3$-$C_6H_4$-$SO_3$- | OH | H |
| 2b | XVIII | $CH_3$ | OH | $pCH_3$-$C_6H_4$-$SO_3$- | H |
| 2b | XIX | $CH_3$ | $pCH_3$-$C_6H_4$-$SO_3$- | $pCH_3$-$C_6H_4$-$SO_3$- | H |
| 3a | XX | $CH_3$ | $CH_3SO_3$- | $CH_3SO_3$- | CBZ |
| 3a | XXI | $CH_3$ | $CH_3SO_3$- | OH | CBZ |
| 3a | XXV | $CH_3$ | OH | $CH_3SO_3$- | CBZ |
| 3b | XXIII | $CH_3$ | $CH_3SO_3$- | OH | H |
| 3b | XXIV | $CH_3$ | OH | $CH_3SO_3$- | H |
| 4 | XXV | $CH_3$ | $(PhO)_2PO_2$ | OH | CBZ |
| 4 | XXVI | $CH_3$ | $(PhO)_2PO_2$ | OH | H |
| 5 | XXVII | $CH_3$ | $NaSO_3$- | OH | H |
| 5 | XXVIII | $CH_3$ | OH | $NaSO_3$- | H |

*CBZ = benzyloxycarbonyl; Bz = benzyl.

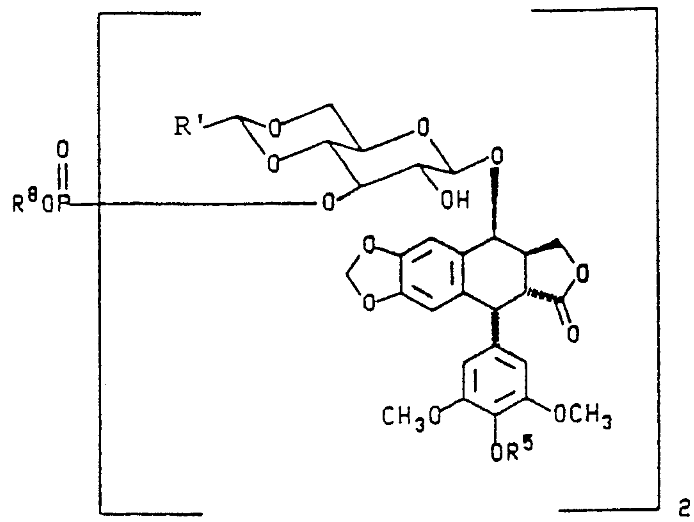

VI

| Ex | Compound | $R^1$ | $R^5$ | $R^8$ |
|---|---|---|---|---|
| 1b | IX | $CH_3$ | CBZ | Bz |
| 1c | XIII | $CH_3$ | H | Na- |

Example 6

The general procedures described in Examples 1 to 5 are repeated using teniposide instead of etoposide to provide the teniposide analogs of compounds VII - XXVIII (i.e. $R^1$ is 2-thienyl).

## Claims

1. A compound having the formula

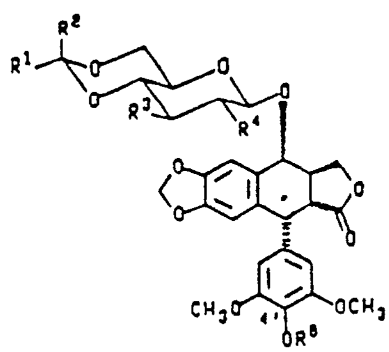

wherein
$R^2$ is H and $R^1$ is selected from the group consisting of $(C_{1-10})$alkyl; $(C_{2-10})$alkenyl; $(C_{5-6})$cycloalkyl; 2-furyl; 2-thienyl; $(C_{6-10})$aryl; $(C_{7-14})$aralkyl; and $(C_{8-14})$aralkenyl wherein each of the aromatic rings may be unsubstituted or substituted with one or more groups selected from halo, $(C_{1-8})$alkyl, $(C_{1-8})$alkoxy, hydroxy, nitro, and amino; or $R^1$ and $R^2$ are each $(C_{1-8})$alkyl; or $R^1$ and $R^2$ and the carbon to which they are attached join to

form a $(C_{5-6})$ cycloalkyl group:
$R^3$ and $R^4$ are selected from the group consisting of OH, $-OSO_2R^6$, $-OSO_3H$ and pharmaceutically acceptable salts thereof, $-OP(X)(OH)_2$ and their pharmaceutically acceptable salts, and $-OP(X)(OR^7)_2$, wherein $R^6$ is hydrogen, $(C_{1-5})$alkyl, phenyl, $(C_{1-5})$alkyl-substituted phenyl, or phenyl$(C_{1-5})$alkyl; $R^7$ is a phosphate protecting group; X is oxygen or sulfur; with the proviso that $R^3$ and $R^4$ are not both OH; and
$R^5$ is H or a phenol protecting group.

2. A compound having the formula

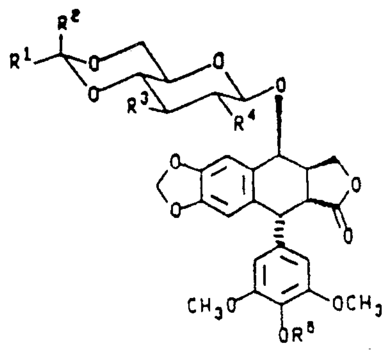

wherein
$R^2$ is H and R' is selected from the group consisting of $(C_{1-10})$alkyl; $(C_{2-10})$alkenyl; $(C_{5-6})$cycloalkyl; 2-furyl; 2-thienyl; $(C_{6-10})$aryl; $(C_{7-14})$aralkyl; and $(C_{8-14})$aralkenyl wherein each of the aromatic rings may be unsubstituted or substituted with one or more groups selected from halo, $(C_{1-8})$alkyl, $(C_{1-8})$alkoxy hydroxy, nitro, and amino; or R' and $R^2$ are each $(C_{1-8})$alkyl; or R' and $R^2$ and the carbon to which they are attached join to form a $(C_{5-6})$ cycloalkyl group;
$R^3$ and $R^4$ are selected from the group consisting of OH, $-OSO_2R^6$, $-OP(X)(OH)_2$ and their pharmaceutically acceptable salts, and $-OP(x)(OR^7)_2$, wherein $R^6$ is hydrogen, $(C_{1-5})$alkyl, phenyl, $(C_{1-5})$alkyl-substituted phenyl, or phenyl $(C_{1-5})$ alkyl; $R^7$ is a phosphate protecting group; X is oxygen or sulfur; with the proviso that $R^3$.and $R^4$ are not both OH; and
$R^5$ is H or a phenol protecting group.

3. A compound of Claim 1 wherein $R^2$ is H and $R^1$ is 2-thienyl.

4. A compound of Claim 2 wherein $R^2$ is H and $R^1$ is 2-thienyl

5. A compound of Claim 2 wherein $R^2$ is H and $R^1$ is methyl.

6. A compound of Claim 5 wherein $R^3$ and $R^4$ are independently selected from the group consisting of OH, $-OSO_2R^6$, and $-OP(X)(OR^7)_2$ wherein $R^6$, $R^7$, and X are as previously defined with the proviso that $R^3$ and $R^4$ are not both OH.

7. A compound of Claim 5 wherein $R^3$ and $R^4$ are independently OH or $-OP(X)(OH)_2$ wherein X is as defined above with the proviso that $R^3$ and $R^4$ are not both OH; or a pharmaceutically acceptable salt thereof.

8. A compound of Claim 5 wherein $R^3$ is OH, $R^5$ is H, and $R^4$ is $-OP(O)(OH)_2$; or a pharmaceutically acceptable salt thereof.

9. A compound of Claim 5 wherein $R^3$ is OH, $R^5$ is H, and $R^3$ is $-OP(O)(OH)_2$; or a pharmaceutically acceptable salt thereof.

10. The disodium salt of the compound of Claim 8.

11. The disodium salt of the compound of Claim 9.

12. A compound of Claim 6 wherein $R^7$ is selected from the group consisting of $(C_{1-5})$alkyl optionally substituted with one or more halogen atom; phenyl optionally substituted with one or more groups selected from $(C_{1-5})$alkyl, halo, and nitro; and phenyl$(C_{1-5})$alkyl wherein the phenyl ring is optionally substituted with one or more groups selected from $(C_{1-5})$alkyl, halo, and nitro.

13. A compound of Claim 12 wherein $R^5$ is benzyloxycarbonyl.

14. A compound of Claim 12 wherein $R^5$ is H.

15. A compound of Claim 14 wherein $R^5$ is OH.

16. A compound of Claim 15 wherein $R^3$ is $-OSO_2$-O-(p-tolyl).

17. A compound of Claim 15 wherein $R^3$ is $-OSO_2-O-CH_3$.

18. A compound of Claim 15 wherein $R^3$ is $-OP(O)(O-C_6H_5)_2$.

19. A compound of Claim 14 wherein $R^3$ is OH.

20. A compound of Claim 15 wherein $R^4$ is $-OSO_2-O-$(p-tolyl).

21. A compound of Claim 15 wherein $R^4$ is $-OSO_2-O-CH_3$.

22. A compound of Claim 15 wherein $R^4$ is $-OP(O)(O-C_6H_5)_2$.

23. A compound of Claim 1 wherein $R^2$ and $R^5$ are each H, $R^1$ is methyl, $R^3$ is $-OSO_3H$, and $R^4$ is OH; or a pharmaceutically acceptable salt thereof.

24. A compound of Claim 1 wherein $R^2$ and $R^5$ are each H, $R^1$ is methyl, $R^4$ is $-OSO_3H$, and $R^3$ is OH; or a pharmaceutically acceptable salt thereof.

25. A compound of Claim 3 wherein $R^2$ and $R^5$ are each H, $R^1$ is 2-thienyl, $R^3$ is $-OSO_3H$, and $R^4$ is OH, or a pharmaceutically acceptable salt thereof.

26. A compound of Claim 3 wherein $R^2$ and $R^5$ are each H, $R^1$ is 2-thienyl, $R^4$ is $-OSO_3H$, and $R^3$ is OH; or a pharmaceutically acceptable salt thereof.

27. A compound having the formula

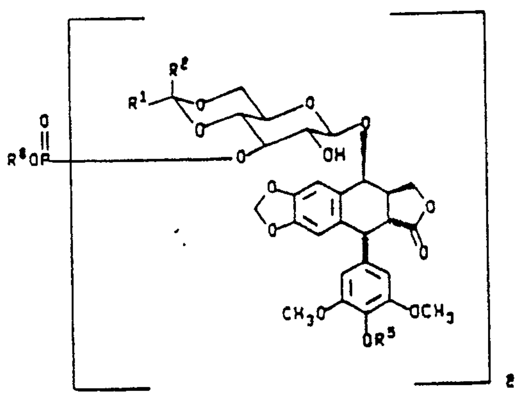

wherein $R^8$ is H or a pharmaceutically acceptable salt thereof, or $R^7$; $R^1$, $R^2$, $R^5$, and $R^7$ are as defined in Claim 1.

28. A compound of Claim 27 wherein $R^1$ is methyl, $R^2$, $R^5$, and $R^8$ are H: or a pharmaceutically acceptable salt thereof.

29. The sodium salt of the compound of Claim 28.

30. The use of at least one compound of anyone of Claims 1 to 29 for preparing a pharmaceutical composition for inhibiting tumor growth in a mammalian host.

31. A pharmaceutical composition which comprises an antitumor effective amount of at least one compound of anyone of Claims 1 to 29 and a pharmaceutically acceptable carrier.

32. A process for preparing the pharmaceutical composition of Claim 31 which comprises incorporating an anti-tumor effective amount of at least one compound of anyone of claims 1 to 29 into a pharmaceutically acceptable carrier.

33. A process for preparing a compound of Claim 1 which comprises the steps of:

(a) protecting the 4′-phenol group of a compound having the formula

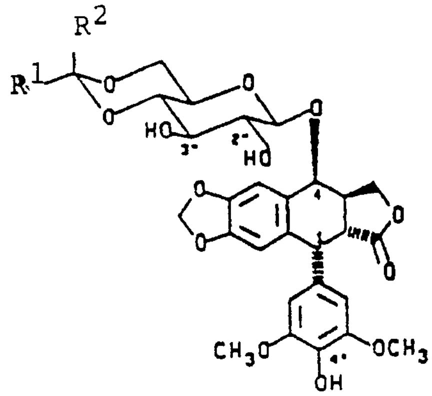

I

(b) reacting the 4′-phenol protected product of Step (a) with a compound selected from the group consisting of $SO_3$, $R^6SO_2Cl$, and $(R^7O)P(X)Cl$ wherein $R^6$, $R^7$, and X are as defined in Claim 1;

(c) optionally removing the phosphate protecting group $R^7$;

(d) optionally removing the 4′-phenol protecting group; and

(e) separating the isomers.

34. A process for preparing a compound of Claim 27 or a compound of Claim 1 wherein $R^3$ and $R^4$ are selected from the group consisting of OH, $-OP(X)(OH)_2$ and their pharmaceutically acceptable salt, and $-OP(X)(OR^7)_2$ wherein X and $R^7$ are as defined in Claim 1, with the proviso that $R^3$ and $R^4$ are not both OH; which comprises the steps of:

(a) protecting the 4′-phenol group of a compound having the formula

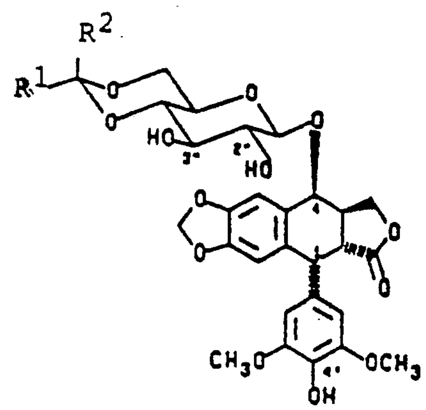

wherein $R^1$ and $R^2$ are as defined in Claim 1;

(b) reacting the 4′-phenol protected product of Step (a) with $POCl_3$ and treating the intermediates generated in situ with an alcohol $R^7OH$;

(c) optionally removing the phosphate protecting group $R^7$;

(d) optionally removing the 4′-phenol protecting group; and

(e) separating the isomers.